# FASCICULE DE BREVET EUROPEEN

(11) **EP 3 374 022 B1**
(45) Date de publication et mention de la délivrance du brevet: **14.08.2019**
(21) Numéro de dépôt: 16794274.7
(22) Date de dépôt: 07.11.2016
(51) Int. Cl.: A61N 5/06, A61B 18/18, A61B 18/00

(54) **PIÈCE À MAIN POUR DISPOSITIF IPL COMPORTANT UN BLOC OPTIQUE MIS EN PLACE PAR PIVOTEMENT**
HANDSTÜCK FÜR EINE IPL-VORRICHTUNG MIT EINER OPTISCHEN, AN IHREN PLATZ GESCHWENKTEN EINHEIT
HANDPIECE FOR AN IPL DEVICE COMPRISING AN OPTICAL UNIT PIVOTED INTO PLACE

(30) Priorité: 13.11.2015 FR 1560924
(43) Date de publication de la demande: 19.09.2018
(73) Titulaire: Eurofeedback, 91017 Evry Cedex (FR)
(72) Inventeur: SAFRAOUI, Georges, 91140 Villejust (FR)
(74) Mandataire: Nony
(86) Numéro de dépôt international: PCT/EP2016/076824
(87) Numéro de publication internationale: WO 2017/080949

(56) Documents cités:
- WO-A1-2008/012519
- WO-A1-2008/050261

## Description

La présente invention concerne les appareils de traitement par émission de flashs lumineux.

De tels appareils sont encore appelés dispositifs de traitement IPL (intense pulsed light) et comportent classiquement une pièce à main que manipule l'opérateur pendant le traitement, reliée par un cordon à un poste de base. La pièce à main loge une cartouche contenant une lampe flash qui est refroidie par une circulation de fluide, en l'espèce le plus souvent de l'eau. Le poste de base comporte un générateur électrique et un circuit électronique de contrôle.

Par exemple, WO 2008 050 261 décrit une pièce à main pour dispositif de traitement par émission de flashs lumineux comportant une cartouche à lampe flash pourvue de connecteur électrique et un bloc optique définissant un logement de réception de la cartouche.

Les cartouches existantes comportent un connecteur fluidique à raccords rapides, comportant des embouts qui s'enfichent dans des logements correspondants selon un mouvement de coulissement lors de la mise en place de la cartouche dans la base. L'effort à exercer peut être relativement important, et l'opération difficile à effectuer pour une personne ne disposant pas de la force nécessaire. De plus, ces embouts portent des joints toriques qui sont introduits profondément dans les logements de la base. Avec la chaleur, ces joints peuvent rester collés dans les logements et se détacher des embouts, créant des difficultés ensuite lors de la mise en place d'une nouvelle cartouche.

Les pièces à main à cartouches sont ainsi relativement encombrantes et il existe un besoin pour améliorer encore l'ergonomie du changement de cartouche et la fiabilité de la connexion fluidique vis-à-vis du problème de collage des joints avec la température.

Par ailleurs, il est souhaitable que le changement de cartouche puisse se faire facilement, tout en garantissant le bon établissement de la connexion électrique et fluidique, et la sécurité de l'opérateur.

En effet, les normes de sécurité médicales (IEC 60601-1) imposent une distance de sécurité suffisante par rapport tout conducteur potentiellement dangereux. Ceci même en cas de premier défaut, comme le stipule le paragraphe 8.1.b de la norme. Ce paragraphe interdit de fait les connexions de type piston dont les bornes restent accessibles après le démontage de la cartouche, car la protection par ouverture de la chaîne de sécurité peut ne pas fonctionner et dans ce cas l'opérateur qui fait le changement peut être en danger, même si le câble secteur est débranché, du fait des énergies électriques stockées. Un test par un doigt artificiel doit être fait pour s'assurer du respect de la norme EN60601-1.

Un autre problème rencontré avec les appareils existants concerne la désionisation de l'eau. Les cartouches de désionisation de l'eau de refroidissement sont en général installées dans le poste de base, et nécessitent un remplacement régulier, soit par l'opérateur, soit par le personnel de maintenance. Le problème est que souvent, par oubli ou négligence, ces cartouches ne sont pas changées, ce qui entraîne une pollution du circuit hydraulique par des micro-organismes (algues, bactéries...), nuisible au bon fonctionnement de l'appareil.

Enfin, la lampe flash doit, pour s'allumer, recevoir une impulsion d'allumage (dite trigger) de très haute tension, typiquement de l'ordre de 12 kV et d'une durée de 1 µs.

Dans les cartouches connues, cette impulsion est apportée par un fil électrique relié à un bouclier thermique métallique faisant écran entre la lumière émise par la lampe et les pièces du boîtier en matière plastique de la cartouche. La présence de ce fil implique la réalisation de connexions étanches, qui compliquent la fabrication de la cartouche.

L'invention vise à répondre à tout ou partie des problèmes identifiés ci-dessus,

.L'invention définie dans les revendications. D'autres modes de réalisation sont simplement exemplaire.

### Montage du bloc optique

L'invention a pour objet, selon un premier de ses aspects, une pièce à main pour appareil de traitement par émission de flashs lumineux, comportant :
- une base,
- une cartouche à lampe flash pourvue de connecteurs fluidique et électrique,
- un bloc optique à fixer sur la base, définissant un logement de réception de la cartouche à lampe flash, le bloc optique étant agencé pour appliquer, au terme d'un mouvement de pivotement, le connecteur fluidique de la cartouche contre un connecteur correspondant prévu sur la base, de façon à établir une circulation de fluide de refroidissement entre la cartouche et la base.

Grâce à l'invention, selon ce premier aspect, une connexion fluidique sans fuite est garantie, car le mouvement de pivotement du capot par rapport à la base permet de presser fortement le connecteur fluidique de la cartouche contre celui de la base.

La mise en place par pivotement permet de gagner en compacité, car les longueurs d'engagement des connecteurs peuvent être raccourcies par rapport à un montage conventionnel par coulissement.

On peut également améliorer l'ergonomie et diminuer l'effort à exercer au changement de cartouche.

Il est possible de faire en sorte que l'on puisse bénéficier dans cette action d'un effet de levier, ce qui est particulièrement intéressant lorsque l'un au moins des connecteurs comporte des joints qui sont écrasés lors du montage.

L'écrasement de ces joints peut nécessiter une force importante, et un effet de levier avec des joints proches de l'axe de rotation permet un effet de multiplication de la force appliquée par l'opérateur.

L'invention permet aussi, selon cet aspect, de gagner en fiabilité vis-à-vis d'un éventuel collage des joints, puisqu'en cas de collage les joints demeurent facilement accessibles.

Le montage de l'ensemble constitué par le bloc optique et la cartouche peut se faire avec une certaine tolérance mécanique, car l'alignement des pièces optiques, qui nécessite de la précision, a lieu lors du montage de la cartouche sur le bloc optique, avant fixation de l'ensemble sur la base de la pièce à main.

Le connecteur fluidique de la base peut comporter deux logements pour recevoir des embouts mâles du connecteur fluidique de la cartouche.

Le bloc optique peut comporter, du côté opposé à l'articulation sur la base, des pattes d'accrochage, et la base peut comporter un bouton de déverrouillage pour libérer les pattes après encliquetage sur la base.

La pièce à main peut avoir une forme générale ronde, lorsque le bloc optique est refermé.

### Connecteur électrique à fente

L'invention a encore pour objet, selon un autre de ses aspects, une pièce à main comportant une base munie d'un connecteur électrique à fente destiné à coopérer avec un connecteur plat correspondant d'une cartouche à lampe flash.

Il est particulièrement avantageux que la base comporte un connecteur électrique à fente pour recevoir un connecteur plat correspondant de la cartouche. La fente permet d'empêcher tout contact accidentel de l'opérateur avec des contacts électriques de la pièce à main lorsque cette dernière est ouverte pour le remplacement de la cartouche. On bénéficie d'une sécurité accrue en garantissant une distance de sécurité suffisante lorsque la pièce à main est ouverte.

Cet aspect de l'invention est indépendant du pivotement du bloc optique sur la base lors de son montage.

Toutefois, le mouvement de rotation de la cartouche est possible en utilisant des contacts à pincement pour la base, qui laissent glisser les parties conductrices du connecteur plat.

De préférence, le connecteur fluidique est plus proche de l'axe d'articulation sur la base que le connecteur électrique. Cela permet de bénéficier d'un effet de levier accru, comme expliqué plus haut.

### Cartouche à résine échangeuse d'ions

L'invention a encore pour objet, selon un troisième aspect, une cartouche de lampe flash qui se caractérise par le fait qu'elle comporte une résine échangeuse d'ions au contact du liquide de refroidissement circulant dans la lampe.

Cette résine garantit la pureté de l'eau utilisée pour refroidir la lampe flash et son remplacement intervient avec celui de la cartouche. Ainsi, les défauts de remplacement de la résine, parfois rencontrés dans l'état de la technique, sont évités. La cartouche optique étant remplacée régulièrement, la résine sera renouvelée par la même occasion et maintiendra ses qualités à l'eau de refroidissement. Le changement de la cartouche optique (consommable) étant obligatoire, la qualité de l'eau sera ainsi maintenue d'une façon certaine au bon niveau. Le circuit hydraulique sera protégé en continu.

### Allumage par couplage capacitif

L'invention a encore pour objet, selon un quatrième de ses aspects, une cartouche pour pièce à main d'appareil de traitement par émission de flashs lumineux, comportant :
- une lampe flash disposée dans une enceinte étanche définie au moins partiellement par une paroi isolante électriquement,
- un contact de connexion électrique à un générateur d'impulsion d'allumage, et une plage conductrice reliée audit contact et venant à proximité de la lampe pour induire à travers ladite paroi isolante, par couplage capacitif, l'ionisation du gaz contenu dans la lampe conduisant à l'allumage de la lampe.

Cela évite les traversées étanches de l'art antérieur et simplifie grandement la fabrication de la cartouche.

L'invention a encore pour objet une cartouche pour une pièce à main telle que définie plus haut, comportant un connecteur fluidique et un connecteur électrique plat. Le connecteur électrique plat est avantageusement défini par un circuit imprimé.

Le bloc optique selon l'un des premier, deuxième, troisième ou quatrième aspects de l'invention peut comporter des reliefs d'actionnement de microcontacts présents sur la base. Cela permet une reconnaissance automatique du bloc optique par l'appareil.

L'invention a encore pour objet un ensemble comportant une pièce à main selon l'invention et une cartouche selon l'invention, reçue dans la pièce à main.

L'invention a encore pour objet un appareil de traitement par émission de flashs lumineux, comportant une pièce à main selon l'invention ou un ensemble bloc optique et cartouche selon l'invention, et un poste de base auquel est relié la pièce à main.

L'invention a encore pour objet un procédé de remplacement d'une cartouche à lampe flash d'un ensemble selon l'invention, comportant les étapes suivantes :
- ouvrir la pièce à main,
- retirer le bloc optique avec la cartouche,
- extraire la cartouche du bloc optique, notamment en s'aidant de la nouvelle cartouche comme d'un outil,
- mettre en place la nouvelle cartouche dans le bloc optique,
- refermer la pièce à main en faisant pivoter l'ensemble bloc optique et cartouche, le mouvement de pivotement amenant le connecteur fluidique de la cartouche à être pressé contre le connecteur fluidique de la pièce à main.

Des caractéristiques avantageuses de l'invention sont énumérées ci-dessous, pouvant être prises isolément ou en combinaison, et s'appliquant à chacun des quatre aspects de l'invention :
- la base comporte un connecteur électrique à fente pour recevoir un connecteur plat correspondant de la cartouche ;
- le connecteur fluidique est plus proche d'un axe d'articulation du bloc optique sur la base que le connecteur électrique à fente ;
- le connecteur fluidique de la base comporte deux logements pour recevoir des embouts mâles du connecteur fluidique de la cartouche ;
- le bloc optique comporte, du côté opposé à l'articulation, des pattes d'accrochage, et la base comporte un bouton de déverrouillage pour libérer les pattes après encliquetage sur la base ;
- la cartouche comporte un premier moyen d'accrochage et la base un deuxième moyen d'accrochage coopérant avec le premier pour permettre de faire pivoter l'ensemble constitué par le bloc optique et la cartouche relativement à la base ;
- les premier et deuxième moyens d'accrochage comportent des crochets ;
- le connecteur fluidique de la cartouche comporte des joints d'étanchéité disposés de façon à être écrasés par la mise en place de la cartouche sur la base :
- la cartouche est fixée par encliquetage sur le bloc optique ;
- le connecteur à fente comporte des contacts à lames flexibles venant s'appliquer sur le connecteur plat de la cartouche ;
- les lames flexibles appartiennent à des contacts en forme de lyre ;
- les contacts portant les lames flexibles sont supportés par deux cartes de circuit imprimé définissant entre elles un espace où peut s'engager le connecteur plat de la cartouche ;
- la base comporte un micro-interrupteur actionné par le connecteur plat de la cartouche au terme de son insertion dans la base ;
- le connecteur plat est constitué par un circuit imprimé double face, des contacts étant définis par des pistes conductrices opposées reliées entre elles par des trous métallisés ;
- le connecteur plat est constitué par un circuit imprimé pourvu d'une découpe définissant une partie flexible pouvant fléchir perpendiculairement à son plan, encliquetée dans un support correspondant de la cartouche ;
- le connecteur plat est défini par un circuit imprimé portant une mémoire électronique renseignant sur l'historique de fonctionnement de la cartouche ;
- la cartouche comporte une coque extérieure et un support fixé dans la coque extérieure, la résine échangeuse d'ions étant reçue dans un logement du support ;
- le logement est délimité par une nervure ménageant un passage pour l'eau à une extrémité du logement et une sortie à l'autre extrémité ;
- la résine échangeuse d'ions est contenue dans un sachet ;
- un orifice communique avec un embout d'un connecteur fluidique débouchant dans le fond du support et de la cartouche ;
- le logement est agencé relativement à l'orifice de telle sorte qu'une fraction seulement de l'eau circule le long de la résine, de préférence moins de 50 % du débit, mieux entre 10 et 30 % ;
- la cartouche comporte un connecteur fluidique comportant deux embouts de connexion hydraulique, seul l'un d'entre eux étant équipé d'un clapet anti-retour, fermé en l'absence de circulation d'eau dans la cartouche ;
- la résine est située entre une paroi de fond et un bouclier formant écran thermique ;
- le contact servant à l'allumage de la lampe est défini par deux pistes conductrices opposées reliées par au moins un trou métallisé ;
- la plage conductrice se superpose à la paroi d'un bouclier formant écran métallique, le long duquel la lampe s'étend ;
- le circuit imprimé porte des contacts de puissance et des contacts reliés à une mémoire électronique ;
- le circuit imprimé comporte une découpe définissant une partie flexible pouvant fléchir perpendiculairement à son plan, encliquetée dans un support de la cartouche.

L'invention a encore pour objet la cartouche considérée isolément, et la pièce à main comportant une telle cartouche, ainsi qu'un appareil de traitement comportant la pièce à main.

L'invention pourra être mieux comprise à la lecture de la description qui va suivre, d'un exemple de mise en oeuvre non limitatif de celle-ci, et à l'examen du dessin annexé, sur lequel :

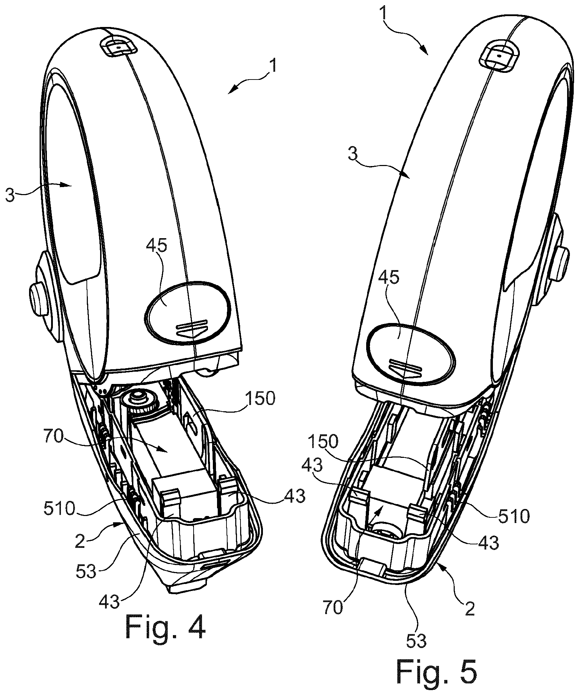

- les figures 1 à 5 représentent, en perspective, la pièce à main sous différents angles de vue,
- les figures 6 et 7 sont deux vues en perspective de la pièce à main après enlèvement d'une demi-coque de la base,
- la figure 8 représente isolément le bloc optique avec la cartouche montée à l'intérieur,
- la figure 9 représente le bloc optique, capot enlevé, avec la cartouche en place,
- les figures 10 et 11 représentent isolément en perspective la cartouche, sous deux angles de vue différents,
- la figure 12 représente la cartouche après enlèvement d'une partie du boîtier de celle-ci,
- les figures 13 et 14 illustrent sous différents angles de vue l'agencement de la lampe flash et de différents autres éléments constitutifs disposés à l'intérieur de la cartouche,
- la figure 15 représente isolément le support de la cartouche,
- la figure 16 représente isolément le circuit imprimé de la cartouche,
- la figure 17 représente en perspective un contact en forme générale de lyre,
- la figure 18 illustre la présence d'un sachet contenant une résine échangeuse d'ions à l'intérieur de la cartouche,
- la figure 19 représente isolément la base, avant mise en place du bloc optique 3,
- la figure 20 représente isolément la base avec enlèvement d'une demi-coquille du boîtier de la base, et

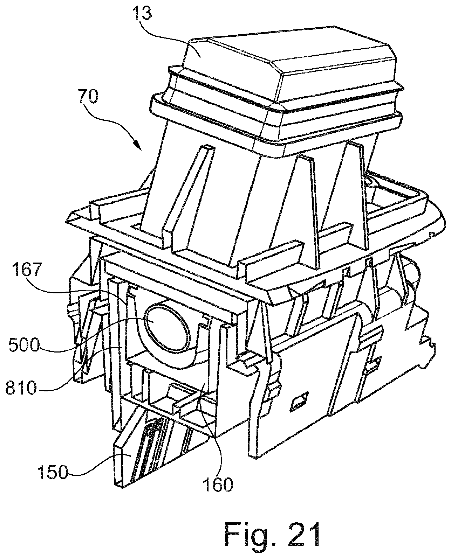

- la figure 21 est une coupe transversale partielle de la cartouche.

La pièce à main 1 représentée sur les figures comporte une base 2 et un bloc optique 3 logeant une cartouche de lampe flash 70, encore appelée cassette.

La base 2 comporte une ouverture 4 définissant une poignée 6.

Un bouton 5 est prévu sur la poignée 6 pour permettre à l'utilisateur tenant celle-ci de déclencher l'émission de flashs lumineux en appuyant dessus avec l'index.

La pièce à main 1 est reliée à un poste de base, non représenté, par un cordon également non représenté, quittant la base 2 par un guide 7. Le cordon loge des tuyaux d'amenée et de retour de liquide de refroidissement, en l'espèce de l'eau, et des câbles électriques.

Le bloc optique 3 comporte un guide optique 13 ayant une face d'extrémité 55, encore appelée face de sortie, par laquelle la lumière quittant la pièce à main est émise. Un capot 53 assure la protection du guide optique 13 et de la cartouche 70.

Le guide 13 traverse la paroi du capot 53 à la faveur d'une ouverture 14, de telle sorte que la face de sortie 55 soit située à l'avant de la pièce à main et puisse être pressée sur la peau.

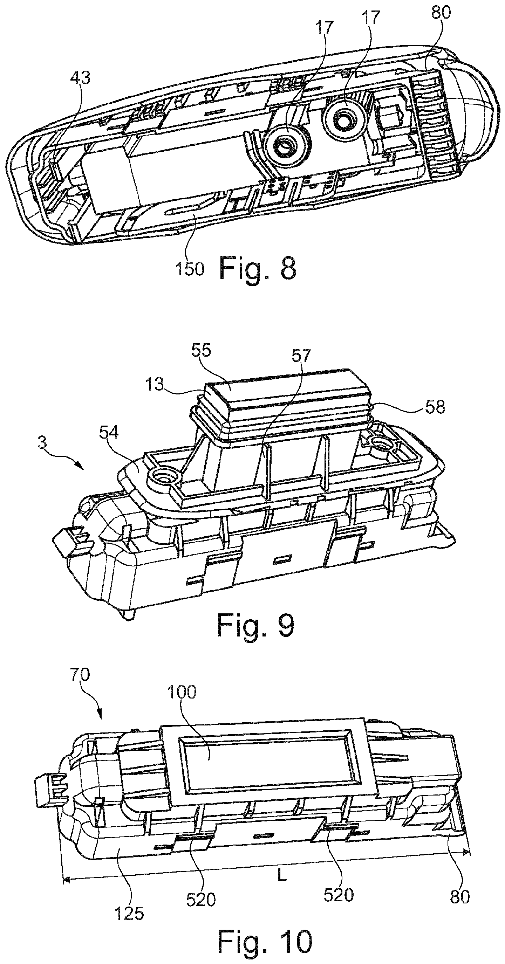

Le bloc optique 3 a été représenté isolément sur la figure 9 avec le capot 53 enlevé. On voit que le bloc optique 3 comporte un corps 54 qui comporte des nervures 57 de maintien du guide 13. Un joint de sortie 58 s'interpose entre le guide 13 et l'ouverture 14 du bloc optique 3.

La cartouche 70 est représentée isolément à la figure 10. On voit qu'elle présente une fenêtre de sortie 100, qui est positionnée dans le bloc optique 3 de telle sorte qu'elle vienne en regard de la face d'entrée du guide 13, et de préférence à son contact.

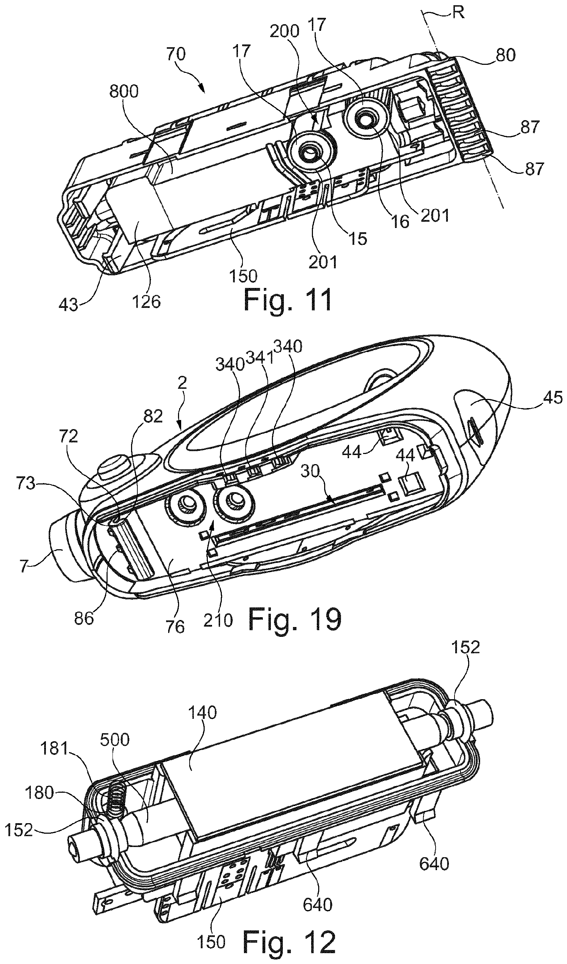

La cartouche 70 comporte, comme on peut le voir sur la figure 11 notamment, un connecteur fluidique 200 à deux embouts 15 et 16, parallèles l'un à l'autre, et un connecteur électrique plat 150, constitué dans l'exemple décrit par une carte de circuit imprimé.

Dans l'exemple considéré, le connecteur 200 comporte des joints toriques 17 reçus chacun dans une gorge annulaire 201 autour de l'embout correspondant 15 ou 16.

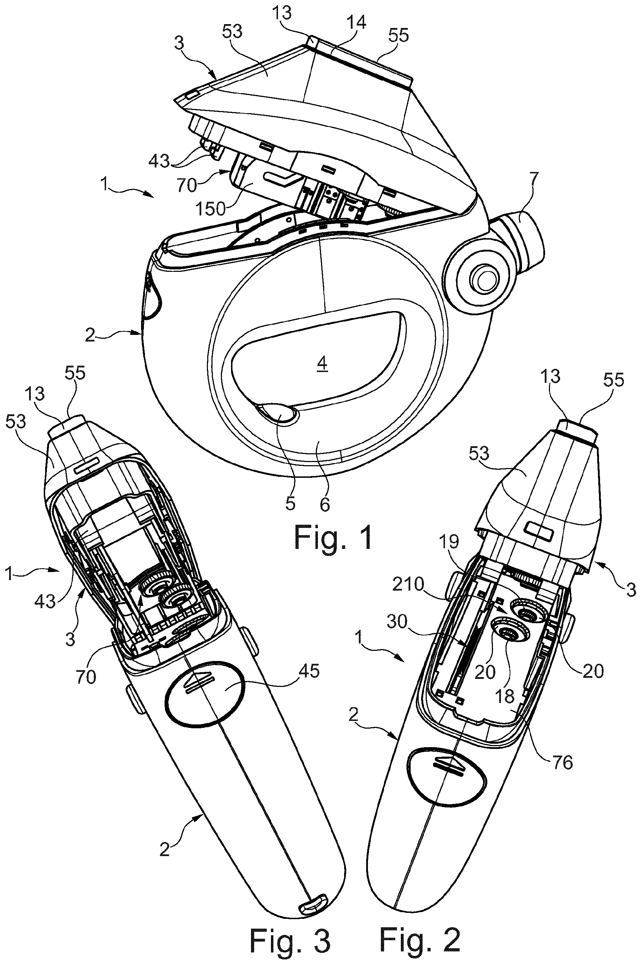

La base 2 comporte, comme visible notamment à la figure 2, un connecteur fluidique 210 comportant deux logements 18 et 19 réalisés en creux dans une paroi 76 et dans lesquels s'engagent respectivement les embouts 15 et 16. Chaque logement 18 ou 19 définit un siège 20 contre lequel s'applique le joint 17 correspondant quand le bloc optique 3 est en position fermée, après avoir été rabattu avec la cartouche 70 sur la base 2.

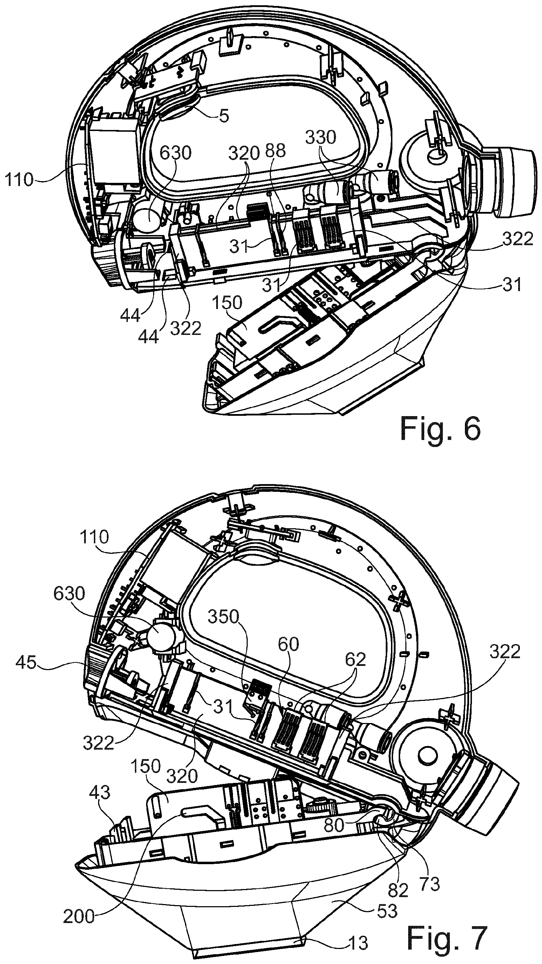

Les joints 17 sont ainsi écrasés entre la cartouche 70 et la base 2 et une bonne étanchéité peut être obtenue. L'écrasement des joints 17 est facilité par un effet de levier obtenu par accrochage de la cartouche 70 sur la base 2, comme illustré sur les figures 6 et 7 notamment.

Plus particulièrement, la base 2 peut comporter comme illustré sur la figure 19 une gorge 72 définie entre un crochet 73 et la paroi 76, et la cartouche 70 un crochet complémentaire 80 destiné à coopérer avec le crochet 73 pour guider le pivotement de la cartouche 70 vers la base 2. Le crochet 80 présente une concavité cylindrique autour d'un axe R, qui est confondu avec l'axe de la tête 82, également cylindrique, du crochet 73, de telle sorte que le mouvement de l'ensemble constitué par le bloc optique 3 et la cartouche 70 lorsque celui-ci est rabattu contre la base 2 est sensiblement un mouvement de rotation autour de l'axe R. Le crochet 73 peut être renforcé, du côté opposé à la gorge 72, par des nervures 86. De même, le crochet 80 de la cartouche 70 peut être renforcé par des nervures 87.

Il est préférable, comme illustré, que le connecteur fluidique 200 de la cartouche 70 soit positionné à proximité de l'axe d'articulation R. Les axes des embouts 15 et 16 sont ainsi positionnés dans l'exemple considéré à une distance de l'axe R qui est inférieure à la demi-longueur L/2 de la cartouche, L désignant la longueur hors tout de la cartouche 70, mesurée le long de son axe longitudinal.

La carte de circuit imprimé 150 constituant le connecteur plat de la cartouche porte des pistes conductrices qui définissent des contacts électriques. Plus particulièrement, dans l'exemple considéré, les contacts sont formés chacun sur les deux faces opposées du circuit imprimé grâce à des trous métallisés 300 qui relient les pistes superposées. Plusieurs trous métallisés 300 peuvent être associés aux deux faces d'un même contact.

La carte 150 porte deux contacts de puissance 301, 302, qui peuvent supporter plusieurs centaines d'ampères, par exemple autour de 300A, deux contacts de mémoire 303, 304 reliés à une puce électronique 600 et un contact de déclenchement 305 relié à une piste étendu 610.

La base 2 comporte un connecteur électrique à fente 30, dans laquelle s'engage le connecteur plat 150 de la cartouche quand le bloc optique 3 est en position fermée.

Des lames conductrices flexibles 31 font saillie à l'intérieur du connecteur à fente 30 pour s'appliquer sur les contacts électriques présents sur les deux faces du circuit imprimé 150.

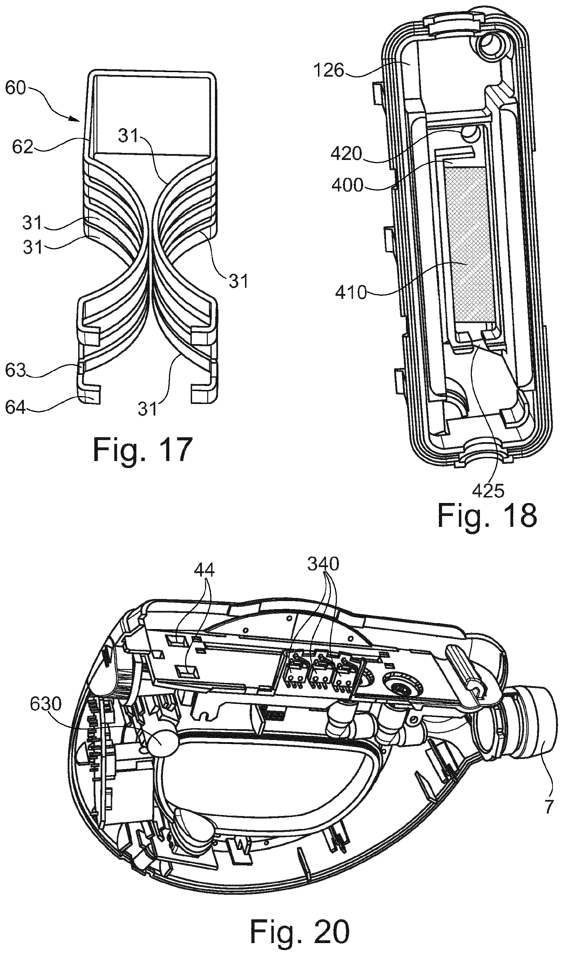

Ces lames 31 appartiennent à des contacts 60 en forme de lyre, dont l'un d'eux est représenté isolément sur la figure 17.

Chaque contact 60 comporte au moins une paire de lames flexibles 31 destinées à pincer les pistes opposées d'un même contact du circuit imprimé 15. Dans l'exemple considéré, quatre paires de lames 31 sont utilisées par contact 60 pour coopérer avec les pistes des contacts de puissance 301 et 302. Les lames 31 d'une même paire sont reliées par une bande large 62, celle-ci étant doublement pliée de façon à positionner les lames 31 d'une même paire en face l'une de l'autre avec un écartement inférieur à l'épaisseur du circuit imprimé 150.

Les lames 31 situées d'un même côté de l'espace de réception du circuit imprimé 150 sont reliées entre elles à leur extrémité 63 opposée à la bande large 62, et les lames 31 les plus extérieures sont prolongées par des retours 64 vers l'intérieur, participant au maintien du contact 60.

La base 2 peut comporter trois contacts 60 à une seule paire de lames 31, pour le signal de déclenchement et la communication avec la mémoire électronique 600 de la cartouche 70.

Chacun des contacts 60 est réalisé par découpe et pliage d'une feuille métallique conductrice, de préférence dorée.

A l'intérieur de la base 2, les contacts 60 sont supportés, comme on peut le voir sur la figure 6, par deux circuits imprimés 320 placés en vis-à-vis, avec des découpes 88 dans lesquelles les lames 31 sont engagées, afin de s'étendre dans l'espace entre les deux circuits imprimés 320 pour pincer le circuit imprimé 150. Chaque contact 60 est positionné à cheval sur les cartes 320 et fixé sur celles-ci par soudure. Les circuits 320 sont maintenus à leurs extrémités opposées par des colonnes 322 pourvues de rainures parallèles dans lesquelles s'engagent les circuits imprimés 320. Les retours 64 des contacts 60 sont soudés sur les cartes 320.

Des raccords coudés 330 visibles sur la figure 6 relient le connecteur fluidique 210 de la base 2 aux tuyaux correspondants du cordon précité.

Quand le bloc optique 3 est en position ouverte, comme illustré sur la figure 1, tout risque de contact de l'opérateur avec les lames flexibles 31 est empêché par l'étroitesse de la fente du connecteur à fente 30, ce qui garantit la sécurité de l'opérateur dans l'éventualité d'une présence de haute tension sur celles-ci.

Le bloc optique 3 peut être maintenu dans la position fermée par encliquetage de deux pattes 43 dans des logements correspondants 44 de la base, apparents sur la figure 6 notamment. Un bouton de déverrouillage 45 permet, lorsque pressé, de libérer les pattes 43 et d'ouvrir la pièce à main 1.

La base 2 comporte un système de reconnaissance de bloc optique 3 comportant des micro-interrupteurs 340 et 341, visibles sur la figure 20. Les micro-interrupteurs 340 peuvent être actionnés par un ou plusieurs reliefs correspondants du bloc optique 3, non apparents, ce ou ces reliefs pouvant varier d'un bloc optique à un autre de façon à ne pas agir sur les mêmes micro-interrupteurs 340.

La base 2 peut ainsi reconnaître selon les reliefs employés plusieurs types de blocs optiques 3 différents. Ces derniers peuvent différer par exemple par le type de guide optique 13 utilisé, par exemple de section plus ou moins grande en extrémité pour concentrer plus ou moins la lumière.

Le micro-interrupteur 341 peut être utilisé pour détecter automatiquement la fermeture du bloc optique 3.

La base 2 comporte un micro-interrupteur 350 positionné entre les deux circuits imprimés 320, visible sur la figure 7, sur lequel vient appuyer le circuit imprimé 150 de la cartouche 70 au terme de son montage. Cela permet de vérifier automatiquement la présence de la cartouche 70 sur la base 2 avant d'autoriser le fonctionnement de l'appareil.

La base 2 peut comporter, comme illustré, un circuit électronique 110 de contrôle et un transformateur 630 permettant de générer la haute tension nécessaire à l'amorçage de la lampe. Ce transformateur 630 peut être reçu dans un logement spécifique de la base 2, de façon à gagner encore en compacité.

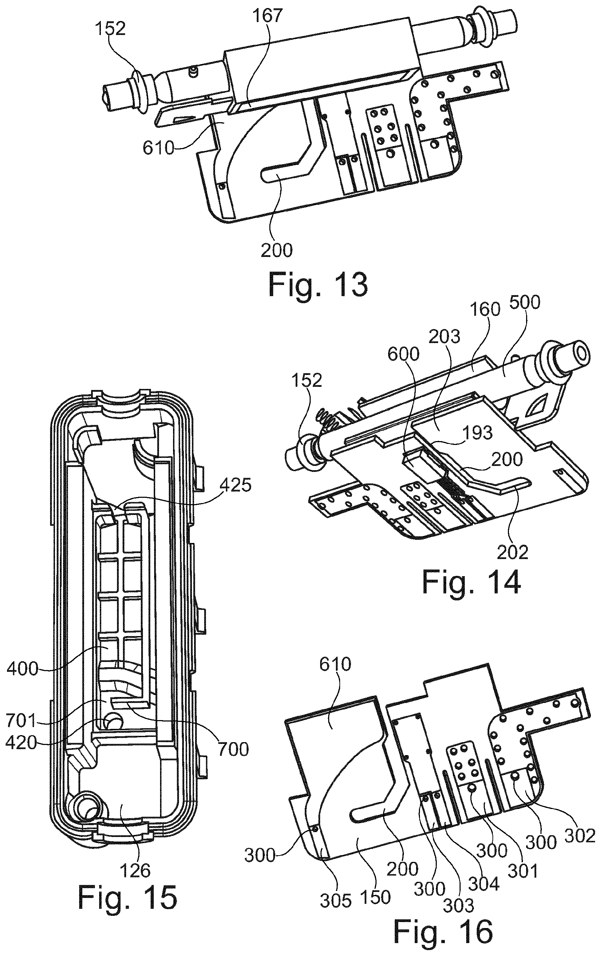

La cartouche 70 est formée par l'assemblage d'une coque extérieure 125 et d'un support 126, représenté isolément à la figure 15, fixé à l'intérieur de la coque 125.

La cartouche définit une enceinte 800 à l'intérieur de laquelle la lampe s'étend, refroidie par eau.

La coque 125 est réalisée avec les pattes d'accrochage 43 et le crochet 80. Le support 126 est réalisé avec les embouts du connecteur fluidique 200 de la cartouche 70. Il porte un filtre optique 140, visible sur la figure 12, constituant la fenêtre de sortie 110 de la cartouche 70, et une lampe flash 150. Des joints toriques 152 sont montés sur les extrémités de la lampe 150. Les électrodes métalliques de la lampe sont refroidies par l'eau, qui vient à leur contact.

Un réflecteur 160 est disposé derrière la lampe 150, comme illustré sur la figure 14. Le réflecteur 160 est placé dans une pièce métallique formant écran 167, à l'intérieur de l'enceinte 800.

Le support 126 porte des pattes 640 servant à sa fixation dans la coque extérieure 125. Cette dernière présente des reliefs 520, visibles sur la figure 10, sur lesquels s'encliquettent des pattes 510 du bloc optique 3, pour retenir la cartouche sur celui-ci.

Le fond du support 126 définit à l'aide d'une nervure 700 un logement 400 pour recevoir un sachet 410 de résine échangeuse d'ions, apparent sur la figure 18, afin de dé-ioniser l'eau lorsqu'elle circule dans la cartouche 70.

L'embout 15 débouche à côté du logement 400 par une ouverture 420.

Ainsi, une partie de l'eau est dirigée directement vers les pièces à refroidir tandis qu'une fraction de l'eau circule par un passage 701 ménagé entre la nervure et la paroi opposée, le long du sachet 410, jusqu'à une sortie 425 du côté opposé au passage 701. Ainsi, seule une fraction de l'eau est envoyée sur la résine, de préférence une fraction comprise entre 10 et 30% du débit.

Un clapet à bille 180, rappelée par un ressort 181, visible sur la figure 12, équipe l'autre embout 15 relié à l'arrivée d'eau. Ce clapet s'ouvre sous la pression de l'eau durant le fonctionnement de l'appareil.

La fermeture du clapet 180, 181 en l'absence de circulation d'eau permet de limiter l'écoulement d'eau hors de la cartouche 70 lors de son remplacement.

La fixation du circuit imprimé 150 sur le support 126 s'effectue par encliquetage d'une dent 193 formant saillie dans une découpe 200 du circuit imprimé 150, sur un relief correspondant du support 126, non apparent.

La découpe 200 se prolonge en formant un coude 202 de façon à conférer une certaine flexibilité à la partie 203 du circuit imprimé 150 portant la dent 193, en flexion dans une direction perpendiculaire à son plan, pour permettre l'encliquetage précité.

La piste étendue 610 constitue une plage conductrice qui se superpose au bouclier métallique 167 et permet, par couplage capacitif, de transmettre à travers une paroi isolante 810 définissant partiellement l'enceinte 800 dans laquelle la lampe est placée, l'impulsion haute tension à la lampe pour ioniser le gaz contenu à l'intérieur et provoquer son allumage.

Le remplacement de la cartouche 70 s'effectue de la façon suivante.

On commence par enlever la cartouche 70 en place avec le bloc optique 3 en appuyant sur le bouton de déverrouillage 45, ce qui libère les pattes 43 et permet à l'ensemble de pivoter pour désengager les crochets 73 et 80.

Ensuite, la cartouche 70 est désolidarisée du bloc optique 3 en s'aidant de la nouvelle cartouche comme d'un outil pour déformer manuellement, localement, la paroi latérale de la coque 125, sur laquelle les pattes 510 du bloc optique 3 sont accrochées, pour les libérer.

La nouvelle cartouche 70 est mise en place dans le bloc optique 3 jusqu'à encliquetage des pattes 510 sur les reliefs correspondants 520.

L'insertion de la cartouche 70 dans le bloc optique 3 permet de positionner la fenêtre de sortie 100 précisément devant la face d'entrée du guide 13.

Ensuite, le crochet 73 est engagé dans le crochet 80 et l'ensemble constitué par le bloc optique 3 et la cartouche 70 fixée à l'intérieur est pivoté jusqu'à l'accrochage des pattes 43.

Au cours de cette opération, les joints 17 sont écrasés, ce qui garantit l'étanchéité de la liaison hydraulique. Le circuit imprimé 150 s'engage entre les lames 31 des contacts 60. Le mouvement d'engagement, qui n'est pas une pure translation, ainsi que les pistes double face du circuit imprimé 150, garantissent une excellente fiabilité de la liaison électrique, malgré l'intensité du courant parcourant les contacts de puissance.

## Revendications

1. Pièce à main pour dispositif de traitement par émission de flashs lumineux, comportant :
- une base (2),
caractérisé en comportant :
- une cartouche (70) à lampe flash pourvue de connecteurs fluidique (200) et électrique (150),
- un bloc optique (3) à fixer sur la base (2), le bloc optique (3) définissant un logement de réception de la cartouche (70), le bloc optique (3) étant agencé pour appliquer, au terme d'un mouvement de pivotement, le connecteur fluidique (200) de la cartouche contre un connecteur correspondant (210) prévu sur la base, de façon à établir une circulation de fluide de refroidissement entre la cartouche et la base.

2. Pièce à main selon la revendication 1, la base comportant un connecteur électrique à fente (30) pour recevoir un connecteur plat (150) correspondant de la cartouche.

3. Pièce à main selon la revendication 2, le connecteur fluidique (210) étant plus proche d'un axe d'articulation (R) du bloc optique (3) sur la base (2) que le connecteur électrique à fente (30).

4. Pièce à main selon l'une quelconque des revendications précédentes, le connecteur fluidique (210) de la base comportant deux logements (18,19) pour recevoir des embouts mâles (15,16) du connecteur fluidique de la cartouche.

5. Pièce à main selon l'une quelconque des revendications précédentes, le bloc optique (3) comportant, du côté opposé à l'articulation, des pattes d'accrochage (43), et la base (2) comportant un bouton de déverrouillage (45) pour libérer les pattes (43) après encliquetage sur la base.

6. Pièce à main selon l'une quelconque des revendications précédentes, la cartouche comportant un premier moyen d'accrochage et la base un deuxième moyen d'accrochage coopérant avec le premier pour permettre de faire pivoter l'ensemble constitué par le bloc optique et la cartouche relativement à la base.

7. Pièce à main selon la revendication 2, les premier et deuxième moyens d'accrochage comportant des crochets (73,80).

8. Pièce à main selon l'une quelconque des revendications précédentes, le connecteur fluidique de la cartouche comportant des joints d'étanchéité (17) disposés de façon à être écrasés par la mise en place de la cartouche sur la base.

9. Pièce à main selon l'une quelconque des revendications précédentes, la cartouche étant fixée par encliquetage sur le bloc optique.

10. Appareil de traitement par émission de flashs lumineux, comportant une pièce à main telle que définie dans l'une quelconque des revendications 1 à 9.

## Patentansprüche

1. Handstück für eine Vorrichtung zur Behandlung mit intensiv gepulstem Licht, welches aufweist:
- ein Unterteil (2),
**dadurch gekennzeichnet, dass** es aufweist:
- eine Patrone (70) mit Blitzlampe, die mit Fluidverbindern (200) und elektrischen Verbindern (150) versehen ist,
- einen optischen Block (3), der am Unterteil (2) zu befestigen ist, wobei der optische Block (3) eine Aufnahme für die Patrone (70) definiert, wobei der optische Block (3) dafür ausgelegt ist, am Ende einer Schwenkbewegung den Fluidverbinder (200) der Patrone an einen entsprechenden Verbinder (210), der am Unterteil vorgesehen ist, anzusetzen, um so eine Zirkulation von Kühlfluid zwischen der Patrone und dem Unterteil herzustellen.

2. Handstück nach Anspruch 1, wobei das Unterteil einen elektrischen Verbinder mit Schlitz (30) aufweist, um einen entsprechenden Flachstecker (150) der Patrone aufzunehmen.

3. Handstück nach Anspruch 2, wobei der Fluidverbinder (210) einer Gelenkachse (R) der Verbindung des optischen Blockes (3) mit dem Unterteil (2) näher ist als der elektrische Verbinder mit Schlitz (30).

4. Handstück nach einem der vorhergehenden Ansprüche, wobei der Fluidverbinder (210) des Unterteils zwei Aufnahmen (18, 19) aufweist, um Einsteckmuffen (15, 16) des Fluidverbinders der Patrone aufzunehmen.

5. Handstück nach einem der vorhergehenden Ansprüche, wobei der optische Block (3) auf der dem Gelenk gegenüberliegenden Seite Einhaklaschen (43) aufweist und das Unterteil (2) einen Entriegelungsknopf (45) zum Freigeben der Laschen (43) nach dem Einrasten am Unterteil aufweist.

6. Handstück nach einem der vorhergehenden Ansprüche, wobei die Patrone ein erstes Einhakmittel und das Unterteil ein zweites Einhakmittel, das mit dem ersten zusammenwirkt, aufweist, um zu ermöglichen, ein Schwenken der aus dem optischen Block und der Patrone bestehenden Anordnung relativ zum Unterteil zu bewirken.

7. Handstück nach Anspruch 2, wobei das erste und das zweite Einhakmittel Haken (73, 80) aufweisen.

8. Handstück nach einem der vorhergehenden Ansprüche, wobei der Fluidverbinder der Patrone Dichtungen (17) aufweist, die derart angeordnet sind, dass sie durch die Anbringung der Patrone auf dem Unterteil zusammengepresst werden.

9. Handstück nach einem der vorhergehenden Ansprüche, wobei die Patrone durch Einrasten am optischen Block befestigt wird.

10. Vorrichtung zur Behandlung mit intensiv gepulstem Licht, welche ein Handstück aufweist, wie in einem der Ansprüche 1 bis 9 definiert.

## Claims

1. Handpiece for a device for pulsed light treatment, comprising:
- a base (2)
**characterized by** comprising:
- a flash lamp cartridge (70) provided with fluidic (200) and electric (150) connectors,
- an optical unit (3) to be secured to the base (2), the optical unit (3) defining a recess for receiving the cartridge (70), the optical unit (3) being arranged so as to press, at the end of a pivoting movement, the fluidic connector (200) of the cartridge against a corresponding connector (210) provided on the base, so as to establish a flow of cooling fluid between the cartridge and the base.

2. Handpiece according to Claim 1, the base comprising a slot-type electrical connector (30) for receiving a corresponding flat connector (150) of the cartridge.

3. Handpiece according to Claim 2, the fluidic connector (210) being closer to an articulation axis (R) of the optical unit (3) on the base (2) than the slot-type electrical connector (30).

4. Handpiece according to any one of the preceding claims, the fluidic connector (210) of the base comprising two recesses (18, 19) for receiving male end pieces (15, 16) of the fluidic connector of the cartridge.

5. Handpiece according to any one of the preceding claims, the optical unit (3) comprising latching tabs (43) on the side opposite the articulation, and the base (2) comprising an unlocking button (45) for releasing the tabs (43) after snap-fitting onto the base.

6. Handpiece according to any one of the preceding claims, the cartridge comprising a first latching means and the base comprising a second latching means which cooperates with the first latching means such that the assembly consisting of the optical unit and the cartridge can be pivoted relative to the base.

7. Handpiece according to Claim 2, the first and second latching means comprising hooks (73, 80).

8. Handpiece according to any one of the preceding claims, the fluidic connector of the cartridge comprising seals (17) arranged so as to be squashed by the installation of the cartridge on the base.

9. Handpiece according to any one of the preceding claims, the cartridge being secured by snap-fitting on the optical unit.

10. Device for pulsed light treatment, comprising a handpiece as defined in any one of Claims 1 to 9.
